# EUROPEAN PATENT APPLICATION

(11) **EP 2 042 142 A2**
(43) Date of publication of application: **01.04.2009**
(21) Application number: 07254791.2
(22) Date of filing: 12.12.2007
(51) Int. Cl.: A61F 13/472

(54) **Sanitary napkin**

(30) Priority: 27.09.2007 CN 200710161898
(71) Applicant: KANG NA HSIUNG ENTERPRISE CO., LTD., Chia-Li Town T'ai nan Hsien (TW)
(72) Inventor: Tai, Jung-Chi, Tainan Hsien (TW); Yang, Ho-Hsi, Tainan Hsien (TW); Su, Chien-Chung, Chia-Li Town (TW)
(74) Representative: Mounteney, Simon James

(57) **Abstract**

A sanitary napkin (2) includes a flexible absorbing rod body (21), an elastic band (25), and a flap (22). The absorbing rod body (21) has a longitudinal first side (201) adapted to block a vaginal opening, and a longitudinal second side (202) opposite to the first side (201). The elastic band (25) is disposed longitudinally at the second side (202) of the absorbing rod body (21), and applies a contracting force to the second side (202) so that the first side (201) is curved. The flap (22) projects from the second side (202) of the absorbing rod body (21), and is adapted to facilitate handling of the sanitary napkin (2).

## Description

This invention relates to a sanitary napkin, more particularly to a sanitary napkin that is suitable for use when wearing a thong garment.

Women usually use a sanitary panty shield before or after a menstrual period or on days when a small amount of discharge or excretion occurs. However, when a user wears a thong garment, such as a T-back or G-string, or the like, and uses the aforementioned sheet-like panty shield which has a large area, the panty shield is likely to extend past the undergarment and be exposed, and is thus not suitable for use on such a garment.

Therefore, the object of the present invention is to provide a sanitary napkin that can be disposed on an outer portion of a vaginal opening and that has a small volume so as not to extend past an undergarment of a user and thereby be exposed.

According to this invention, a sanitary napkin comprises a flexible absorbing rod body, an elastic band, and a flap. The absorbing rod body has a longitudinal first side adapted to block a vaginal opening, and a longitudinal second side opposite to the first side. The elastic band is disposed longitudinally at the second side of the absorbing rod body, and applies a contracting force to the second side so that the first side is curved. The flap projects from the second side of the absorbing rod body, and is adapted to facilitate handling of the sanitary napkin.

Other features and advantages of the present invention will become apparent in the following detailed description of the preferred embodiment of the invention, with reference to the accompanying drawings, in which:
Fig. 1 is a perspective view of a sanitary napkin according to the preferred embodiment of the present invention;
Fig. 2 is a sectional view of the preferred embodiment;
Fig. 3 is a schematic side view of the preferred embodiment in a state of use; and
Fig. 4 is a sectional view of the preferred embodiment in a state of use.

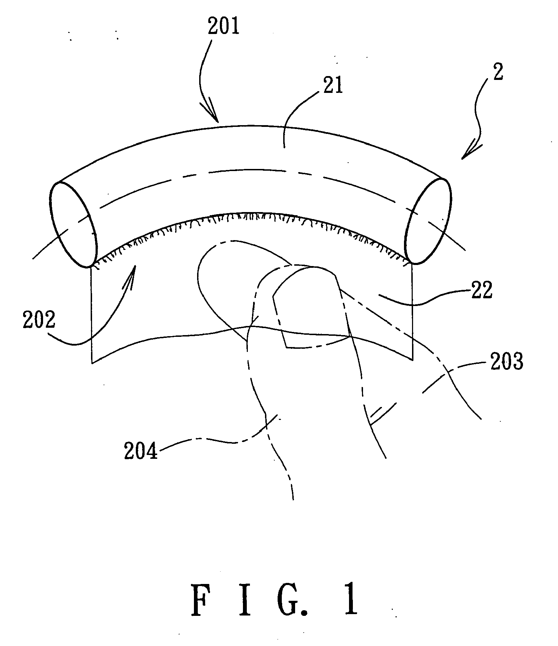

Referring to Figs. 1 to 4, the preferred embodiment of a sanitary napkin 2 according to the present invention is shown to comprise a flexible absorbing rod body 21, a flap 22, and an elastic band 25.

The flexible absorbing rod body 21 has a circular cross-section, a longitudinal first side 201 adapted to block a vaginal opening, and a longitudinal second side 202 opposite to the first side 201. The absorbing rod body 21 includes an absorbing core 24 extending along the length of the absorbing rod body 21, and an outer layer 23 enclosing the absorbing core 24. The absorbing core 24 may be made of a material that is commonly used in an outer layer of a conventional sanitary napkin. The outer layer 23 is made of a non-woven fabric material, and has a longitudinal marginal section 231 extending at the second side 202 of the absorbing rod body 21.

The flap 22 projects from the second side 202 of the absorbing rod body 21, and is connected integrally to the outer layer 23. The flap 22 is adapted to facilitate handling of the sanitary napkin 2.

The elastic band 25 is disposed longitudinally at the second side 202 of the absorbing rod body 21, and is bonded to and is sandwiched between the marginal section 231 of the outer layer 23 and the flap 22 by using an adhesive. The elastic band 25 applies a contracting force to the second side 202, so that the first side 201 of the absorbing rod body 21 is curved. Alternatively, the elastic band 25 may be bonded to the absorbing rod body 21 using other methods, such as a hot-pressing process, an ultrasonic wave process, a high-frequency sealing process, etc.

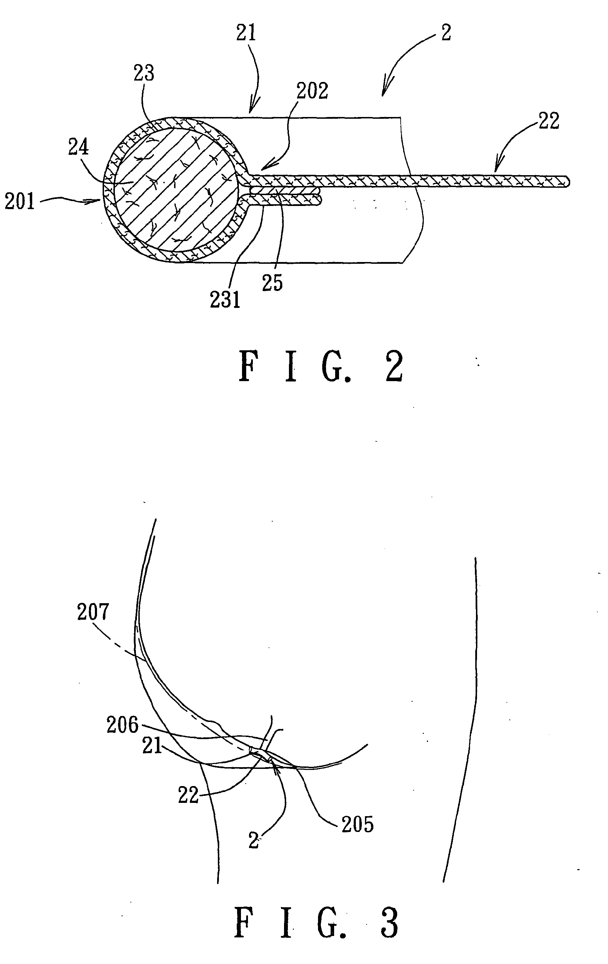

With reference to Figs. 1 and 3, to use the sanitary napkin 2, the user grasps the flap 22 with her fingers 203, 204, and places the first side 201 of the absorbing rod body 21 on an outer portion 205 of her vagina, so that the absorbing rod body 21 is disposed between the labia and blocks the vaginal opening 206. The flap 22 is distal from the vaginal opening 206 at this time. Since the first side 201 curves downwardly due to an applied contraction force of the elastic band 25, the first side 201 contacts more fittingly the outer portion 205 of the vagina. To remove the sanitary napkin 2, the user simply uses her fingers 203, 204 to pull away the sanitary napkin 2 from the outer portion 205 of her vagina through the flap 22.

Additionally, the outer layer 23 may undergo an embossment process so as to soften its material, so that when the absorbing rod body 21 is placed on the outer portion 205 of the vagina, the outer layer 23 does not frictionally rub against the skin of the vaginal area of the user.

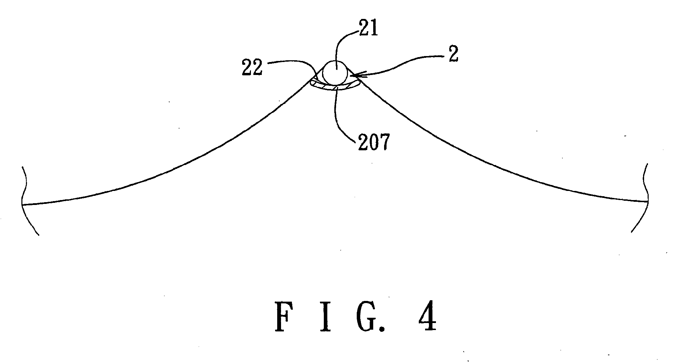

With reference to Figs. 3 and 4, since the volume of the entire sanitary napkin 2 is small, the sanitary napkin 2 is not easily exposed from the undergarment, even when the user wears a thong garment 207.

From the aforementioned description, since the absorbing rod body 21 of the sanitary napkin 2 is located between the labia and blocks the vaginal opening 206 so as to absorb the discharge or excretion from the vagina, the sanitary napkin 2 is easy to use and remove. Further, since the sanitary napkin 2 is disposed on the outer portion 205 of the vagina, and has a small volume, as compared to the conventional sanitary panty shield, even if the user wears a thong garment 207, the sanitary napkin 2 is not likely to extend beyond the garment 207.

## Claims

1. A sanitary napkin (2), **characterized by**:
a flexible absorbing rod body (21) having a longitudinal first side (201) adapted to block a vaginal opening, and a longitudinal second side (202) opposite to said first side (201);
an elastic band (25) disposed longitudinally at said second side (202) of said absorbing rod body (21) and applying a contracting force to said second side (202) so that said first side (201) is curved; and
a flap (22) projecting from said second side (202) of said absorbing rod body (21) and adapted to facilitate handling of said sanitary napkin (2).

2. The sanitary napkin (2) of Claim 1, **characterized in that** said absorbing rod body (21) includes an absorbing core (24) extending along the length of the absorbing rod body (21), and an outer layer (23) enclosing said absorbing core (24).

3. The sanitary napkin (2) of Claim 2, **characterized in that** said outer layer (23) extends around said absorbing core (24) and is connected to said flap (22) at said second side (202), said elastic band (25) being attached to said flap (22) in close proximity to said second side (202).

4. The sanitary napkin (2) of Claim 3, **characterized in that** said outer layer (23) has a longitudinal marginal section (231) extending at said second side (202) and bonded to said elastic band (25) opposite to said flap (22), said elastic band (25) being sandwiched between said marginal section (231) and said flap (22).

5. The sanitary napkin (2) of Claim 2, **characterized in that** said outer layer (23) is made of a non-woven fabric material.
